(19) Europäisches Patentamt
European Patent Office
Office européen des brevets

(11) Publication number: **0 002 852**
**B1**

(12) # EUROPEAN PATENT SPECIFICATION

(45) Date of publication of patent specification: **25.11.81**

(51) Int. Cl.³: **C 07 C 43/30,**
**C 07 C 31/133**

(21) Application number: **78200354.5**

(22) Date of filing: **06.12.78**

(54) **Novel intermediate in the preparation of cyclopropylcarboxylate esters and process for its manufacture.**

(30) Priority: **16.12.77 GB 5246477**

(43) Date of publication of application:
**11.07.79 Bulletin 79/14**

(45) Publication of the grant of the European patent:
**25.11.81 Bulletin 81/47**

(84) Designated Contracting States:
**BE CH DE FR GB IT LU NL**

(56) References cited:
**Chemical Abstracts vol. 68, no. 15 1968 Columbus, Ohio, USA M. MASANAO et al "Selective synthesis of optically pure (+) and (−) transchrysanthemic acids from (+)-delta-3-carene" pages 6597, column 1, abstract no. 68 492w**

**Chemical Abstracts vol. 74, no. 9, 1971 Columbus, Ohio, USA E. WENKERT et al "Cyclopropanol derivatives as intermediates for organochemical synthesis" page 364, column 2, abstract no 42 507d**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Van Berkel, Johannes**
**Badhuisweg 3**
**NL-1031 CM Amsterdam (NL)**
Inventor: **Kelderman, Hendrik Cornelis**
**Badhuisweg 3**
**NL-1031 CM Amsterdam (NL)**

(74) Representative: **Keuzenkamp, Abraham et al,**
**P.O. Box 302**
**NL-2501 CH Den Haag (NL)**

**EP 0 002 852 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Novel intermediate in the preparation of cyclopropylcarboxylate esters and process for its manufacture

The invention relates to compound which is a useful intermediate in the preparation of cyclopropylcarboxylate esters. The invention also relates to a process for the preparation of the compound.

The cyclopropylcarboxylate esters are insecticidally-active compounds known as "pyrethroids" and as they combine exceptionally good insecticidal properties with a very low mammalian toxicity, they are of considerable interest to the agrochemical industry and much effort has been extended in finding economic routes to them and to their principal intermediates.

The general formula of one class of these pyrethroid compounds may be represented as follows:

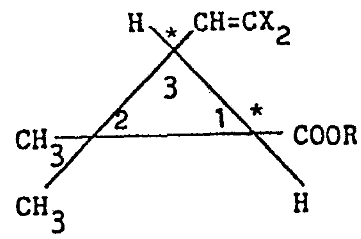

(I)

where each asterisk denotes an asymmetric carbon atom; each X is a halogen atom; and R is a member of a group of radicals known to impart insecticidal activity to the molecule, e.g. 3-phenoxybenzyl or *alpha*-cyano-3-phenoxy-benzyl. It is known that the stereo-isomeric form of the acid portion of the ester of formula I should be in the (IR, cis) form for maximum insecticidal activity, i.e. the absolute configuration at carbon atom I is R and the two hydrogen atoms on carbon atoms 1 and 3 are in a *cis* relationship. This nomenclature is known as the Elliott nomenclature and is defined in M. Elliott, A.W. Farnham, N.F. James, P.H. Needham and D.A. Pullman, Nature, 1974, *248*, 710.

It follows, therefore, that if these stereo isomeric esters of formula I are to be prepared, either a stereospecific chemical route is required or the desired stereoisomer must be obtained from a racemic form by physical separation techniques. The latter are expensive and laborious and not readily employed on an industrial scale. The Applicant has found a stereospecific route which uses as starting material the naturally-occurring substance (+)-3-carene whose formula is as follows:—

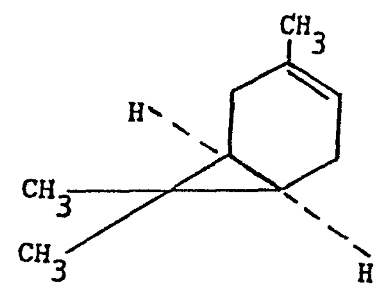

(II)

This compound is an inexpensive readily-available natural terpene and in our copending application European Patent Publication No. 0002850 (K.332) is disclosed a route to the (IR, cis)-acid portion of the pyrethroid ester of formula I starting from (+)-3-carene and proceeding via the novel cyclopropane compound according to the invention.

The present invention provides 3-hydroxymethyl-2,2-dimethylcyclopropanecarbaldehyde dimethyl acetal whose formula is:—

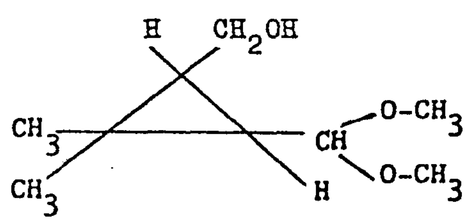

(III)

Preferably the compound of formula III is in the same stereoisomeric form as that of the cyclopropane ring of (+)3-carene because this form leads to compounds of general formula I (R=H) in the (IR, cis) form; such compounds generate optimum levels of insecticidal activity in the pyrethroid end-product.

The compound III or its preferred stereoisomeric form may be prepared by methods known *per se*, for example by hydrolysis of a corresponding ester, see "Methoden der Organischen Chemie" (Houben-Weyl), Volume VIII (1952), 418—423 and 638—639. It has been found that the compound may be conveniently prepared by a method which is characterised in that 3-acetoxymethyl-2,2-dimethylcyclopropanecarbaldehyde dimethyl acetyl of formula:—

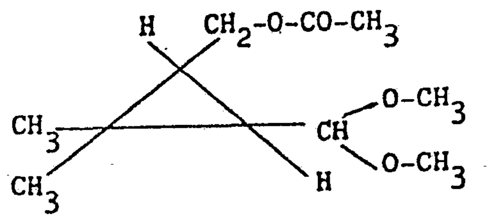

(IV)

is subjected to hydrolysis under conditions which do not affect the dimethoxymethyl group, for example an alkaline-reacting medium, e.g. aqueous sodium or potassium hydroxide. Preferably compound IV is employed in the same stereoisomeric form as that of the cyclopropane ring of (+)-3-carene.

As has been indicated above a multi-stage process for the preparation of insecticidally active compounds, starting with (+)-3-carene and in which the compound according to the invention is employed as intermediate, is described in European Patent Publication No. 0002850.

The following Example further illustrates the invention. Yields and purities were determined by means of gas-liquid chromatography and nuclear magnetic resonance (NMR) spectroscopy. The NMR data quoted were recorded at 90 MHz using solutions of the compounds in deuterochloroform.

*Example — Preparation of a stereoisomer of 3-hydroxymethyl 2,2-dimethylcyclopropanecarbaldehyde dimethyl acetal (compound III)*

(a) *Preparation of a stereoisomer of 1-(2-dimethoxyethyl (-2,2-dimethyl-3-(2-oxo-propyl)cyclopropane (compound A)*

A flask was charged with (+)-3-carene (375 mmol) and waterfree methanol (150 ml) and kept at a temperature of —60°C. Then, a mixture of ozone and oxygen was passed through the liquid in the flask at a rate of 70 l/h (corresponding to 75 mmol of ozone per hour until the (+)-3-carene was fully converted (5 hours). The reaction mixture formed was allowed to adopt a temperature of 20°C, dimethyl sulphide (750 mmol) and p-toluene-sulphonic acid (1.74 mmol) were added and the mixture formed was stirred for four days at 20°C. At the end of this period the (+)-3-carene was fully converted into compound A. Methanol and dimethyl sulphide were evaporated from the reaction mixture, at a pressure of 24 mbar (40°C), diethyl ether (150 ml) was added to the residue obtained, the solution formed was washed with a 5%w aqueous solution of sodium hydrogen carbonate (30 ml), with four 30 ml portions of water, the washed solution was dried over anhydrous magnesium sulphate and the solvent was evaporated from the dried liquid at a temperature of 30°C and a pressure of 24 mbar to give a residue (68.9 g). This residue was distilled at 83°C/l mbar to give a fraction consisting of cis isomer, compound A, yield 73.5%.

(b) *Preparation of a stereoisomer of 2-(2,2-dimethoxyethyl)-3,3-dimethylcyclopropylmethyl acetate (compound B)*

The contents of a flask charged with compound A (200 mmol) prepared as in (a) in this Example, chloroform (300 ml) and 3-chloroperbenzoic acid (384 mmol) were stirred at 20°C for 24 hours. The precipitate formed was separated by filtration, the filtered precipitate was mixed with n-pentane (150 ml), the mixture was separated by filtration, the combined filtrates were washed with two 50 ml portions of a 5% solution of sodium carbonate in water and with two 50 ml portions of water, the washed liquid was dried over anhydrous magnesium sulphate and the solvent was evaporated from the dried liquid at a temperature of 90°C and a pressure of 20 mbar to give a residue containing compound B in a yield of 97%. The content of compound B in the residue was 92%, only the cis isomer had been formed.

(c) *Preparation of a stereoisomer of 2,2-dimethyl-3- (2-formylethyl) cyclopropyl methyl acetate (compound C)*

The contents of a flask charged with compound B (218 mmol) in the residue prepared as described in (b), acetic acid (40 ml) and water (20 ml) were stirred at 60°C during 2.5 hours. The solvent was evaporated from the reaction mixture at a temperature of 45°C and a pressure of 24 mbar, the residue obtained was taken up in diethyl ether (150 ml), the solution obtained was washed with two 50 ml portions of a 5% solution of sodium hydrogen carbonate in water and with two 50 ml portions of water, the washed solution was dried over anhydrous magnesium sulphate and the solvent

was evaporated from the dried liquid at a temperature of 30°C and a pressure of 24 mbar to give a residue containing compound C in a yield of 80%; the content of compound C in the residue was 85%. Only the cis isomer has been formed.

(d) *Preparation of a stereoisomer of 2-(3-acetoxymethyl-2,2-dimethylcyclopropyl)vinyl acetate (compound D)*

The contents of a flask charged with compound C (175 mmol) in the residue prepared as described in (c), triethylamine (386 mmol) and acetic anhydride (350 ml) were stirred at 20°C for 18 hours. The solvent was evaporated from the reaction mixture at a temperature of 70°C and a pressure of 20 mbar, the residue obtained was washed with five 40 ml portions of water, the washed solution was dried over anhydrous magnesium sulphate and the solvent was evaporated from the dried liquid at a temperature of 40°C and a pressure of 20 mbar to give a residue containing compound D in quantitative yield. The content of compound D in the residue was 88.4%; 64% of compound D had the cis structure, 36% the trans structure of the carbon-carbon double bond. The orientation to the cyclopropane ring was still cis.

(e) *Preparation of a stereoisomer of 3-acetoxymethyl-2,2-dimethylcyclopropanecarbaldehyde dimethyl acetal (compound E)*

A flask was charged with compound D (175 mmol) present in the residue obtained in d, waterfree methanol (200 ml) and p-toluenesulphonic acid (1.16 mmol) and kept at a temperature of −65°C. Then, a mixture of ozone and oxygen was passed through the liquid in the flask at a rate of 60 l/h (corresponding to 70 mmol of ozone per hour) until compound D was fully converted (2.5 hours). The reaction mixture formed was allowed to adopt a temperature of 20°C, dimethyl sulphide (350 mmol) was added and the mixture formed was stirred for 17 hours at 20°C. Methanol and dimethyl sulphide were evaporated from the reaction mixture, at a pressure of 16 mbar, diethyl ether (50 ml) was added to the residue obtained and so much of a saturated aqueous solution of sodium bicarbonate was added to the mixture that the pH reached a value of 7. Then, the mixture was washed with three 50-ml portions of water, the washed liquid was dried over anhydrous magnesium sulphate and the solvent was evaporated from the dried liquid at a temperature of 40°C and a pressure of 24 mbar to give a residue (29.6 g) containing compound E (yield between 78%). Only the cis isomer had been formed. The NMR spectrum of compound E showed the following absorptions:—

$\delta = 3.38$ ppm (singlet, C—O—*CH3*)
$\delta = 4.2$ ppm (multiplet, *H*—C—O—$CH_3$)
$\delta = 1.2$ ppm (multiplet, *H*—C—C(H)—$(OCH_3)_2$)
$\delta = 1.18$ ppm (singlet, $H_3$C—C—$CH_3$)
$\delta = 1.18$ ppm (singlet, $H_3C$—C—C$H_3$)
$\delta = 1.1$ ppm (multiplet, *H*—C—$CH_2$)
$\delta = 4.2$ ppm (multiplet, H—C—C$H_2$)
$\delta = 2.1$ ppm (singlet, $H_3$C—C(O)—O—)

(f) *Preparation of a stereoisomer of 3-hydroxymethyl-2,2-dimethylcyclopropanecarbaldehyde dimethyl acetal (compound III)*

A flask was charged with all of the residue obtained in (e), water (75 ml), sodium hydroxide (150 mmol) and acetone [illegible].5 ml) and the liquid obtained was kept under reflux (60°C) for three hours. Then, the acetone and part of the water were evaporated at a pressure of 16 mbar, the residue obtained was extracted with five 50 mol portions of diethyl ether (during the last two extractions so much sodium chloride was added that the aqueous phase was saturated with this salt), the combined five extract phases were dried over anhydrous magnesium sulphate and the solvent was evaporated from the dried liquid at a temperature of 40°C and a pressure of 24 mbar to give a residue containing compound III in a yield of 51%, calculated on starting compound D. The cis content was 70%.

The NMR spectrum of the cis isomer of compound III showed the following absorptions:—

$\delta = 3.32$ ppm (singlet, C—O—C$H_3$)
$\delta = 4.83$ ppm (singlet, *H*—C—O—$CH_3$)
$\delta = 1.53$ ppm (doublet, *H*—C—C(H)—$(OCH_3)_2$)
$\delta = 1.05$ ppm (singlet, $H_3$C—C—$CH_3$)
$\delta = 1.05$ ppm (singlet, $H_3C$—C—C$H_3$)
$\delta = 1.2$ ppm (multiplet, *H*—C—$CH_2$)
$\delta = 3.8$ ppm (doublet, $H_2$C—OH)
$\delta = 4.3$ ppm (singlet, $H_2$C—O*H*)

The stereoisomer of compound III was converted into a stereoisomer of 3-(2,2-dichlorovinyl)-2,2-dimethylcyclopropylcarboxylic acid (Formula I: R = H: X = Cl) and it was found that its stereoisomeric form was the same as the (IR, cis) form of the acid, the known active acid portion of certain pyrethroid esters.

## Claims

1. 3-Hydroxymethyl-2,2-dimethylcyclopropanecarbaldehyde dimethyl acetal.

2. Compound according to claim 1 in the same stereoisomeric form as that of the cyclopropane ring of (+)-3-carene.

3. A process for preparing the compound claimed in claim 1 characterised in that 3-acetoxymethyl-2,2-dimethylcyclopropanecarbaldehyde dimethyl acetal is subjected to hydrolysis under conditions which do not affect the dimethoxymethyl group.

4. A process according to claim 3 characterised in that 3-acetoxymethyl-2,2-dimethylcyclopropanecarbaldehyde dimethyl acetal is employed in the same stereoisomeric form as the cyclopropane ring of (+)-3-carene.

5. A process according to claim 3 or 4 characterised in that the hydrolysis is carried out in an alkaline-reacting medium.

## Patentansprüche

1. 3-Hydroxymethyl-2,2-dimethylcyclopropancarbaldehyd-dimethylacetal.

2. Verbindung nach Anspruch 1, in der gleichen stereoisomeren Form wie diejenige des Cyclopropanringes von (+)-3-Caren.

3. Verfahren zur Herstellung der Verbindung nach Anspruch 1, dadurch gekennzeichnet, dass man 3-Acetoxymethyl-2,2-dimethylcyclopropancarbaldehyd-dimethylacetal unter Bedingungen, bei denen die Dimethoxymethylgruppe nicht angegriffen wird, der Hydrolyse unterwirft.

4. Verfahren nach Anspruch 3, dadurch gekennzeichnet, dass man 3-Acetoxymethyl-2,2-dimethylcyclopropancarbaldehyd-dimethylacetal in der gleichen stereoisomeren Form verwendet, wie sie der Cyclopropanring von (+)-3-Caren aufweist.

5. Verfahren nach Anspruch 3 oder 4, dadurch gekennzeichnet, dass man die Hydrolyse im alkalisch reagierenden Medium durchführt.

## Revendications

1. Acétal diméthylique de 3-hydroxyméthyl-2,2-diméthylcyclopropanecarbaldéhyde.

2. Un composé selon la revendication 1, caractérisé en ce qu'il est dans la même forme stéréoisomère que celle du noyau de cyclopropane du (+)-3-carène.

3. Un procédé pour préparer le composé defini dans la revendication 1, caractérisé en ce qu'on soumet l'acétal diméthylique de 3-acétoxyméthyl-2,2-diméthylcyclopropanecarbaldéhyde à une hydrolyse dans des conditions qui n'affectent pas le groupe diméthoxyméthyle.

4. Un procédé selon la revendication 3, caractérisé en ce qu'on utilise l'acétal diméthylique de 3-acétoxyméthyl-2,2-diméthylcyclopropanecarbaldéhyde dans la même forme stéréoisomère que celle du noyau de cyclopropane du (+)-3-carène.

5. Un procédé selon l'une des revendications 3 et 4, caractérisé en ce qu'on conduit l'hydrolyse dans un milieu à réaction alcaline.